# EUROPEAN PATENT APPLICATION

(11) **EP 1 491 214 A1**
(43) Date of publication of application: **29.12.2004**
(21) Application number: 03708496.9
(22) Date of filing: 05.03.2003
(51) Int. Cl.: A61K 48/00, A61K 45/00, A61K 9/51, A61K 9/08, A61K 38/45, A61P 1/16, A61P 35/00

(54) **DRUGS FOR TREATING LIVER DISEASES WITH THE USE OF HOLLOW PROTEIN NANOPARTICLES**

(30) Priority: 29.03.2002 JP 2002097457
(71) Applicant: Japan Science and Technology Agency, Kawaguchi-shi, Saitama 332-0012 (JP)
(72) Inventor: KURODA, Shunichi, Suita-shi, Osaka 565-0872 (JP); TANIZAWA, Katsuyuki, Toyono-gun, Osaka 563-0214 (JP); KONDO, Akihiko, Kobe-shi, Hyogo 657-0015 (JP); UEDA, Masakazu, Shinjuku-ku, Tokyo 162-0837 (JP); SENO, Masaharu, Okayama-shi, Okayama 703-8273 (JP); IWABUKI, Hidehiko, Onsen-gun, Ehime 791-0303 (JP)
(74) Representative: Bentz, Jean-Paul
(86) International application number: PCT/JP2003/002600
(87) International publication number: WO 2003/082343

(57) **Abstract**

The invention provides a drug for treating hepatic diseases with the use of hollow protein nanoparticles. The effectiveness of the drug has been proved by animal testing. The invention also provides a therapeutic method using such a drug. In a drug according to the present invention, a substance to be transferred into a cell for treating a hepatic disease (for example, a cancer treating gene such as a thymidine kinase gene derived from simple herpes virus) is encapsulated in hollow nanoparticles that have an ability to recognize a hepatocyte and are composed of a particle-forming protein (for example, hepatitis B virus surface-antigen protein).

## Description

### TECHNICAL FIELD

The present invention relates to drugs for treating hepatic diseases with the use of hollow protein nanoparticles. The invention particularly relates to a drug which contains particles encapsulating a substance to be transferred into a cell for treating a hepatic disease, wherein the drug allows the substance to be specifically incorporated into a hepatic cell.

### BACKGROUND ART

In the field of medicine, there has been active research on drugs that directly and effectively act on the affected area without causing serious side effects. One area of active research is a method known as a drug delivery system (DDS), in which active ingredients of drugs or other substances are specifically delivered to a target cell or tissue, where they can exhibit their effects.

Another area of active research is a technique of gene transfer to a specific cell, which is now essential in the field of molecular cell biology. With the genetic background of various diseases being revealed by the Human Genome Project, a method of highly specific gene transfer to a specific cell or tissue holds great promise because, once the method is established, it is applicable to the field of gene therapy.

In one known example of a gene transfer method to cells, uptake of genes takes place in the form of a giant molecule by endocytosis (calcium phosphate method, lipofectamin method). In another method, genes are transferred through cell membrane pores that are formed by the stimulation of the cell membrane with an electrical pulse (electroporation method, gene gun method). Both of these methods are commonly used in molecular biology experiments.

Despite the simplicity of these methods, they cannot be readily applied to cells or tissues of internal body, because the methods involve direct physical contact with the cells and surgically expose the site of gene transfer. It is also difficult to achieve near 100% uptake.

A transfer method that is safe to use is a liposome method. The liposome method does not damage the cell and is applicable to cells or tissues of internal body. A problem of the method, however, is that the liposome, which is a simple lipid, cannot have a high level of specificity to the cells or tissues, and uptake of genes *in vivo* is far below the required level.

In a recently developed technique, a therapeutic gene is inserted in viral DNA, and the gene is transferred by an infectious virus. The method is innovative in the sense that it does not expose the site of transfer, is applicable to individuals, and provides nearly 100% uptake. However, the method suffers from a serious drawback in that the virus non-specifically infects a wide range of cells, transferring the gene to cells other than the target cell. Further, the method has a potential risk of unexpected side effect if the viral genome is incorporated in the chromosomes. In fact, the method is not used in initial stages of disease treatment. Only the terminal patients can receive the benefit of the method.

Under these circumstances, the inventors of the present invention have previously proposed a method of specifically and safely delivering and transferring various substances (including genes, proteins, compounds) into a target cell or tissue, using hollow nanoparticles of a protein that has the ability to form particles and has incorporated a bio-recognizing molecule, as disclosed in International Publication with International Publication No. WO41/64930 (published on September 7, 2001) (hereinafter referred to as "International Publication WO01/64930"). However, these publications do not fully discuss how the method can be used to develop therapeutic drugs for the treatment of diseased organs.

The present invention was made in view of the foregoing problems, and an object of the invention is to provide a drug for treating hepatic diseases with the use of hollow protein nanoparticles, the therapeutic effects of which have been confirmed by animal testing. The invention also provides a therapeutic method using such a drug.

### DISCLOSURE OF INVENTION

The inventors of the present invention accomplished the present invention by finding that, when hepatitis B virus surface-antigen particles encapsulating a hepatic cancer-treating gene were administered through intravenous injection in laboratory animals to which human hepatic cancer had been transplanted, the gene was specifically incorporated into a human liver-derived tissue part, and was effective in the treatment of transplanted cancer.

That is, the present invention discloses a drug (medicament) in which a substance to be transferred into a cell for treating a hepatic disease is encapsulated in hollow nanoparticles that have an ability to recognize a hepatocyte and are composed of a particle-forming protein.

An example of the particle-forming protein is a hepatitis B virus surface-antigen protein. In eukaryotic cells, the protein is expressed as a membrane protein on the endoplasmic reticulum and accumulates thereon before it is released as particles to the lumen side. With the ability to recognize a hepatocyte, the hollow nanoparticles can act as a carrier, delivering the substance encapsulated in the particles specifically to a hepatocyte. Thus, a hepatic disease-treating substance (medicament) may be encapsulated in the thus obtained hollow nanoparticles. This provides an effective therapeutic drug that specifically and effectively acts on hepatic cells.

The target-cell substance encapsulated in the hollow nanoparticles may be a cancer treating gene, for example. When the cancer treating gene encapsulated in the drug is a thymidine kinase (HSV1tk) gene derived from simple herpes virus, ganciclovir is additionally administered, as will be described in Examples.

The present invention discloses a drug that can be used by a convenient method of intravenous injection to effectively treat hepatic diseases. The drug is a great leap forward from conventional hepatic disease treatment methods in that it does not require large dose or any surgical operation in disease treatment including gene therapy, and that the risk of side effect is greatly reduced. The drug is therefore usable in clinical applications in its present form.

The present invention discloses a treatment method for treating hepatic diseases through administration of the drug disclosed in the present invention.

For a fuller understanding of the nature and advantages of the invention, reference should be made to the ensuing detailed description taken in conjunction with the accompanying drawings.

### BRIEF DESCRIPTION OF DRAWINGS

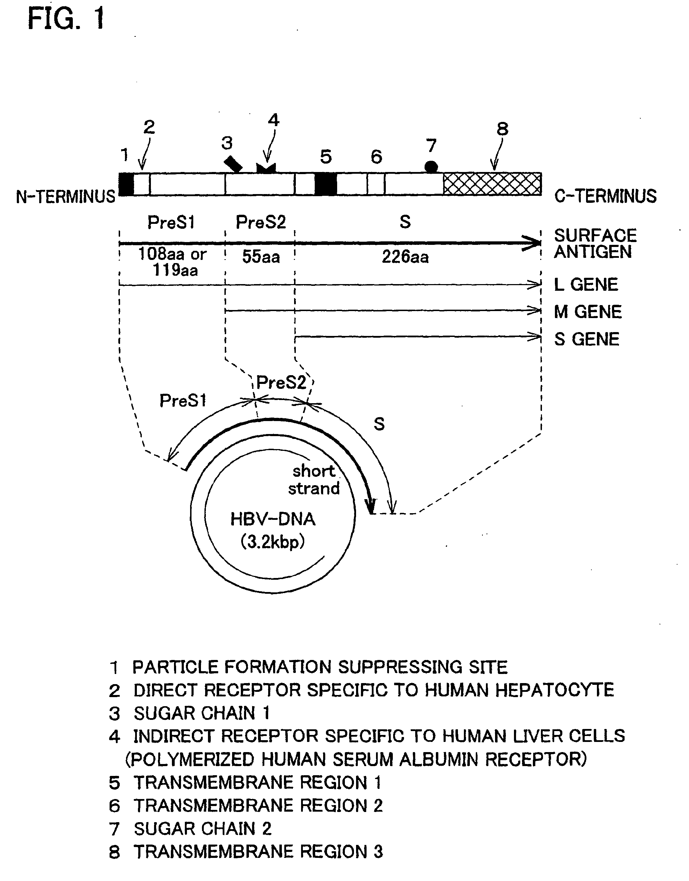

Figure 1 is a schematic drawing showing each protein region of an HBsAg gene in the Examples of the present invention. 1 to 8 indicates the function at each site of the surface antigen. Note that, the number 1 in Figure 1 indicates a particle formation suppressing site. The number 2 indicates a direct receptor specific to human hepatocyte. The number 3 indicates a sugar chain 1. The number 4 indicates an indirect receptor specific to human hepatocyte (polymerized human serum albumin receptor). The number 5 indicates a transmembrane region 1. The number 6 indicates a transmembrane region 2. The number 7 indicates a sugar chain 2. The number 8 indicates a transmembrane region 3.

Figure 2 is an explanatory schematic drawing showing the expression and purification procedures for HBsAg particles using recombinant yeast in an Example of the present invention as an example. Each represents: (a) Preparation of recombinant yeast; (b) Culture on a High-Pi medium; (c) Culture on an 8S5N-P400 medium; (d) Disruption; (e) Density gradient centrifugation; and (f) HBsAg particles.

Figure 3 is a graph showing therapeutic effects of the drug of the present invention on experimental animals.

Figure 4 is a drawing an example of a substance to be transferred to a cell of the present invention.

Figure 5 is a drawing an example of a substance to be transferred to a cell of the present invention.

Figure 6 is a drawing an example of a substance to be transferred to a cell of the present invention.

Figure 7 is a drawing an example of a substance to be transferred to a cell of the present invention.

Figure 8 is a table showing therapeutic effects of the drug of the present invention on experimental animals.

### BEST MODE FOR CARRYING OUT THE INVENTION

Hollow nanoparticles that compose a drug of the present invention allow a substance to be specifically delivered to a hepatic cell or tissue, by inserting a bio-recognizing molecule into its particle-forming protein. The particle-forming protein may be sub viral particles obtained from various viruses. Specific examples of such a protein include hepatitis B virus (HBV) surface-antigen protein.

Particles of such a particle-forming protein may be obtained through the protein expression in the eukaryotic cell. Specifically, in eukaryotic cells, the particle-forming protein is expressed on the endoplasmic reticulum as a membrane protein and accumulates thereon before it is released as particles. The eukaryotic cell may be obtained from yeasts, insects, or animals including mammals.

As will be described later in Examples, the inventors of the present invention have reported that the expression of HBV surface-antigen L protein in recombinant yeast cells produces ellipsoidal hollow particles with a minor axis of 20 nm and a major axis of 150 nm, with a large number of L proteins embedded in the yeast-derived lipid bilayer membrane (J. Biol. Chem., Vol. 267, No. 3, 1953-1961, 1992). The particles contain no HBV genome and lack the viral function. Therefore, the particles are very safe to the human body. In addition, the particles are effective as transporters for specifically transporting substances to hepatic cells, because they display on their surface a hepatocyte specific receptor that bear the high infectivity of HBV to hepatic cells.

Therefore, forming the protein particles using recombinant yeasts offers a preferable method of efficiently producing particles from soluble proteins in the yeasts.

The insect cell, being a eukaryote closer to some of the higher animals than the yeast, is able to form a higher order structure such as a sugar chain unachievable by yeasts. In this connection, the insect cell provides a preferable method of producing heteroproteins in large amounts. The conventional insect cell line used the baculovirus and involved viral expression. This has caused a cell death or lysis in the protein expression. A problem of this method, then, is that the protein expression proceeds continuously, or the proteins are decomposed by the free protease separated from the dead cells. Further, in the secretion and expression of proteins, inclusion of a large amount of fetal bovine serum contained in the culture medium has made it difficult to purify proteins even when proteins are secreted in the medium. In recent years, Invitrogen Corporation has developed and marketed an insect cell line that can be cultured without a serum and without being meditated by the baculovirus. Such an insect line can be used to obtain protein particles that are easy to purify and form into higher order structures.

By modifying the receptor on the surface of particles obtained by the methods as described above into an optional bio-recognizing molecule, or by incorporating various substances (DNAs, RNAs, proteins, peptides, drugs, etc.) into the particles, it is possible to allow hollow protein nanoparticles of the present invention to very specifically deliver and transfer these substances to hepatic cells or hepatic tissues.

The particle-forming protein is not just limited to the hepatitis B virus surface-antigen protein, and may be any protein that is able to form particles. For example, animal cells, plant cells, viruses, natural proteins derived from fungi, and various types of synthetic proteins may be used. Further, when there is a possibility that, for example, virus-derived antigen proteins may trigger antibody reaction in a target organism, a particle-forming protein with suppressed antigenic action may be used as a bio-recognizing molecule. For example, such a particle-forming protein may be the hepatitis B virus surface-antigen protein modified to suppress its antigenic action, or other types of modified proteins (hepatitis B virus surface-antigen protein modified by genetic engineering), as disclosed in International Publication WO01/64930. Other types of proteins such as growth factor and antibody may be further added to the hepatitis B virus surface-antigen protein or its modified proteins.

As the bio-recognizing molecule (molecule that recognizes a hepatic cell or tissue) that is incorporated into a particle-forming protein, for example, cell function-regulating molecules such as growth factor and cytokines; cell or tissue-recognizing molecules such as cell surface antigen, tissue specific antigen, receptor; molecules derived from viruses or microorganisms; antibodies; sugar chains; lipids; and the like may preferably be used.

As described, the present invention provides hollow protein nanoparticles that encapsulate a substance (target-cell substance) to be transferred into a hepatic cell or tissue, and thereby provides a substance carrier having hepatocyte specificity. The substance carrier may encapsulate any substance including, for example, genes in the form of DNA or RNA, natural or synthetic proteins, oligonucleotides, peptides, drugs, and natural or synthetic compounds.

For example, human RNase1 or RNase3 may be used, as previously reported by the inventors of the present invention. Human RNase1 is documented in Jinno H, Ueda M, Ozawa S, Ikeda T, Enomoto K, Psarras K, Kitajima M, Yamada H, Seno M Life Sci. 1996; 58(21): 1901-8. Human RNase3 (also known as ECP (eosinophil cationic protein)) is documented in Mallorqui-Fernandez G, Pous J, Peracaula R, Aymami J, Maeda T, Tada H, Yamada H, Seno M, de Llorens R, Gomis-Ruth FX, Coll M; J Mol Boil. 2000 Jul 28; 300(5): 1297-307.

The proteins have cytotoxicity, the effects of which are both intracellular and extracellular. With the RNase encapsulated in the substance carrier (drug) of the present invention, the cytotoxicity of the protein can be masked outside the cell, and the protein exhibits its effect only inside the cell. It is expected that this will provide a novel cancer treatment method that causes fewer side effects.

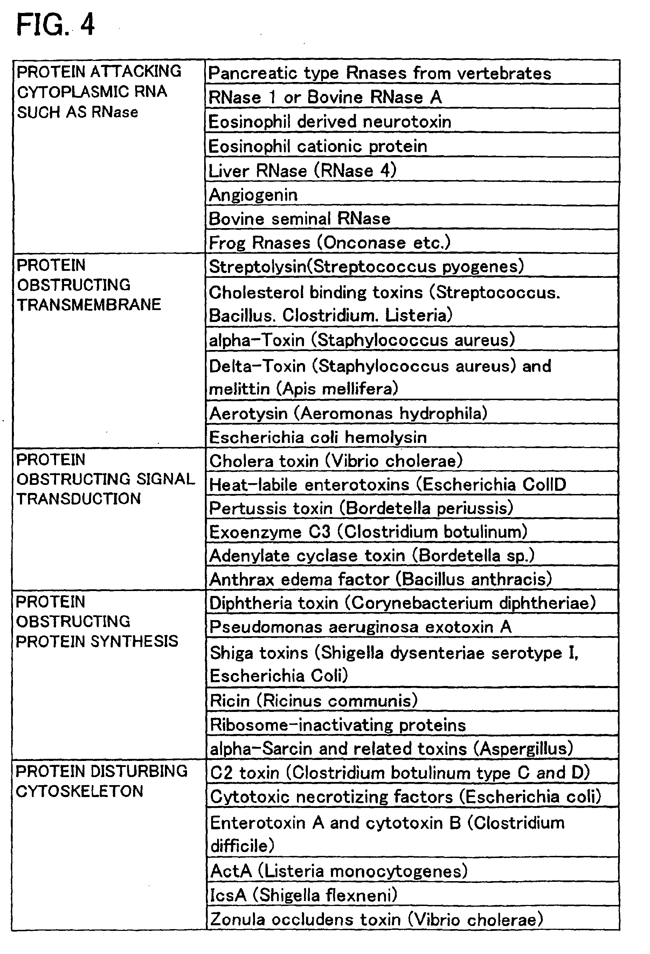

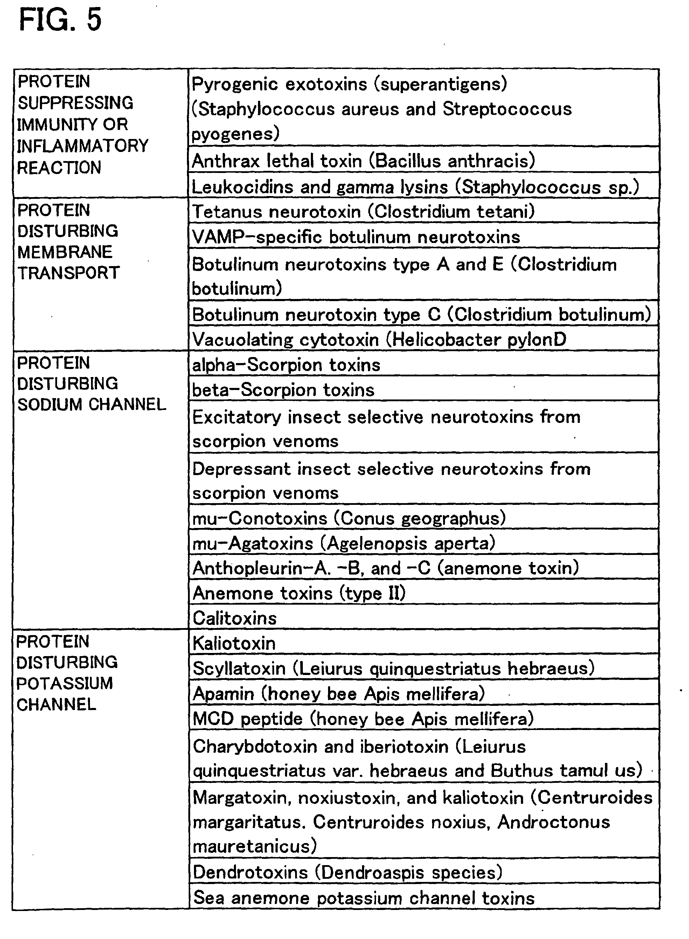

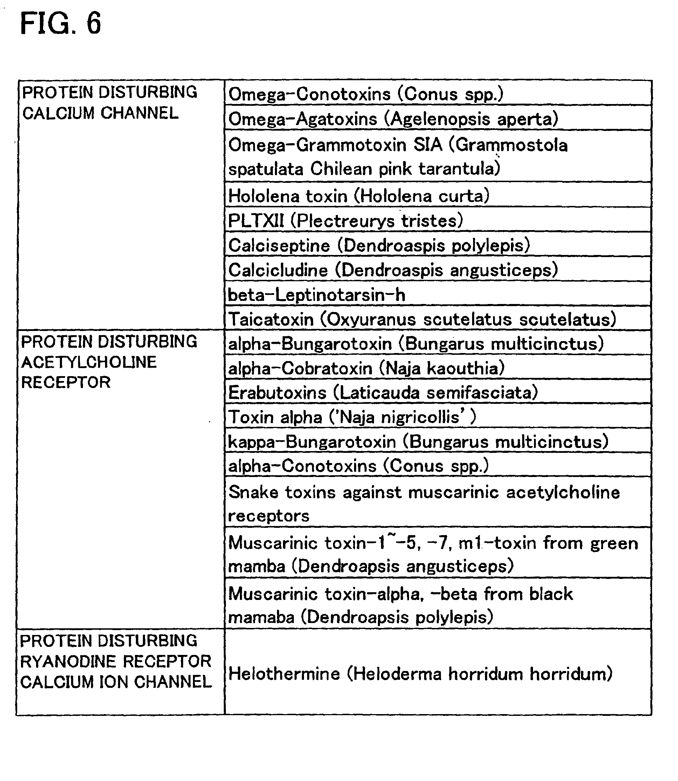

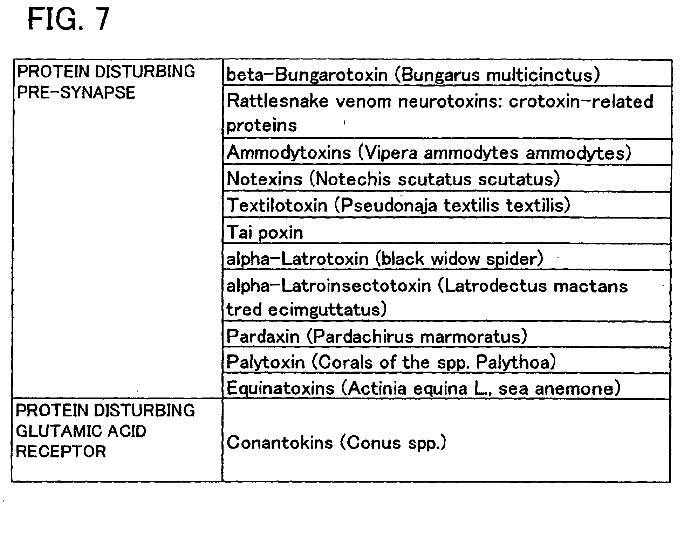

Note that, the target-cell substance may be proteins shown in Fig. 4 through 7, or genes that encode these proteins. Other examples of the substance are various cytokines (various interferons, various interleukins, etc.) that are effective in the treatment of hepatic diseases, and therapeutic genes such as cancer suppressor genes (p53, etc.).

These target-cell substances may be incorporated into the hollow nanoparticles by various methods commonly used in chemical or molecular biological experimental techniques. Some of the preferred examples include an electroporation method, ultrasonic method, simple diffusion method, and a method using charged lipids.

The hollow protein nanoparticles or substance carrier allow the substance to be specifically transported into cells or tissues *in vivo* or *in vitro*. Specific transport of the substance into a specific cell or specific tissue with the use of a drug composed of the hollow protein nanoparticles may be used as a treatment method of various diseases, or one of the steps in the procedure of the treatment method, as in the foregoing example involving the RNase.

The effectiveness of the treatment using the drug of the present invention has been confirmed by animal testing, as will be described later in the Examples. In the Examples, cells derived from human hepatic carcinoma were transplanted in nude rats, and the drug of the present invention and ganciclovir (GCV) were administered to each rat in separate doses. Inside the drug, a thymidine kinase (HSV1tk) gene derived from simple herpes virus was encapsulated. The effectiveness of the treatment was confirmed by observing the size of grafted cancer tissue. The drug was administered intravenously. However, oral administration, intramuscular administration, intraperitoneal administration, subcutaneous administration, or other administration routes are also available.

In the following, the present invention will be described in more detail by way of Examples and Comparative Examples. It should be appreciated that the present invention is not limited in any ways by the following Examples and Comparative Examples.

In the following, HBsAg refers to hepatitis B virus surface antigen. HBsAg is an envelope protein of HBV, and includes three kinds of proteins S, M, and L, as schematically illustrated in Fig. 1. S protein is an important envelope protein common to all three kinds of proteins. M protein includes the entire sequence of the S protein with additional 55 amino acids (pre-S2 peptide) at the N-terminus. L protein contains the entire sequence of the M protein with additional 108 amino acids or 119 amino acids at the N-terminus.

The pre-S regions (pre-S1, pre-S2) of the L protein of HBsAg have important roles in the binding of HBV to the hepatocytes. The Pre-S1 region has a direct binding site for the hepatocytes, and the pre-S2 region has a polymeric albumin receptor that binds to the hepatocytes via polymeric albumin in the blood.

Expression of HBsAg in the eukaryotic cell causes the protein to accumulate as membrane protein on the membrane surface of the endoplasmic reticulum. The L protein molecules of HBsAg agglomerate and are released as particles into the ER lumen, carrying the ER membrane with them as they develop.

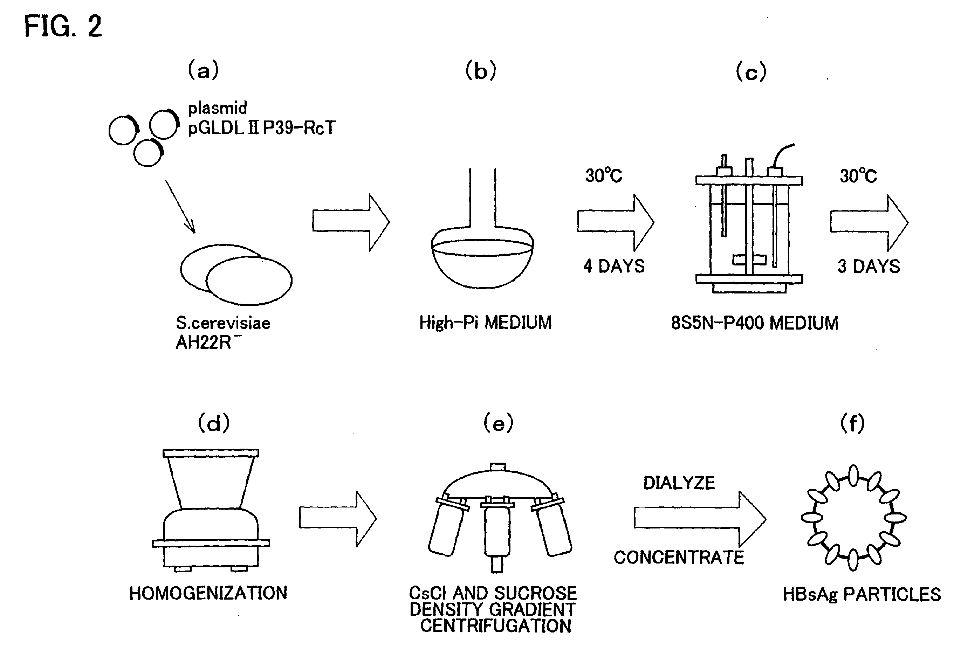

The Examples below used L proteins of HBsAg. Fig. 2 briefly illustrates procedures of expression and purification of HBsAg particles described in the following Examples.

### [Example A] Expression of HBsAg particles in recombinant yeasts

Recombinant yeasts (*Saccharomyces cerevisiae* AH22R-strain) carrying (pGLDLIIP39-RcT) were cultured in synthetic media High-Pi and 8S5N-P400, and HBsAg L protein particles were expressed (Fig. 2a through 2c). The whole procedure was performed according to the method described in J. Biol. Chem., Vol. 267, No. 3, 1953-1961, 1992 reported by the inventors of the present invention.

From the recombinant yeast in stationary growth phase (about 72 hours), the whole cell extract was obtained with the yeast protein extraction reagent (product of Pierce Chemical Co., Ltd.). The sample was then separated by sodium dodecyl sulfate-polyacrylamide gel electrophoresis (SDS-PAGE), and the HBsAg in the sample was identified by silver staining.

The result showed that HBsAg was a protein with a molecular weight of about 52 kDa.

### [Example B) Purification of HBsAg particles from the recombinant yeasts

(1) The recombinant yeast (wet weight of 26 g) cultured in synthetic medium 8S5N-P400 was suspended in 100 ml of buffer A (7.5 M urea, 0.1 M sodium phosphate, pH 7.2, 15 mM EDTA, 2 mM PMSF, and 0.1% Tween 80), and disrupted with glass beads by using a BEAD-BEATER. The supernatant was collected by centrifugation (Fig. 2d).
(2) The supernatant was mixed with a 0.75 volume of PEG 6000 solution (33%, w/w), and cooled on ice for 30 min. The pellets were collected by centrifugation at 7000 rpm for 30 min, and resuspended in buffer A without Tween 80.
(3) The solution was layered onto a 10-40% CsCl gradient, and ultracentrifuged at 28000 rpm for 16 hours. The centrifuged sample was divided into 12 fractions, and each fraction was tested for the presence of HBsAg by Western blotting (the primary antibody was the anti-HBsAg monoclonal antibody). The HBsAg fractions were dialyzed against buffer A without Tween 80.
(4) 12 ml of the dialyzed solution obtained in (3) was layered onto a 5-50% sucrose gradient, and ultracentrifuged at 28000 rpm for 16 hours. As in (3), the centrifuged sample was divided into fractions, and each fraction was tested for the presence of HBsAg. The HBsAg fractions were dialyzed against buffer A containing 0.85% NaCl, without urea or Tween 80 ((2) through (4): Fig. 2e).
(5) By repeating the procedure (4), the dialyzed sample was concentrated with the ultrafilter Q2000 (Advantec), and stored at 4°C for later use (Fig. 2f).

The result of Western blotting after CsCl equilibrium centrifugation in (3) revealed that HBsAg was a protein with S antigenicity with a molecular weight of 52 kDa. At the end of the procedure, about 24 mg of pure HBsAg particles were obtained from the yeast (26 g wet weight) derived from 2.5 L medium.

Each fraction obtained in the purification process was analyzed by SDS-PAGE. In order to confirm whether the purification had successfully removed the yeast-derived protease, the HBsAg particles obtained in (5) were incubated at 37°C for 12 hours, separated by SDS-PAGE, and identified by silver staining.

The result of confirmation showed that the yeast-derived protease had been completely removed by the purification process.

### [Example C] Sealing of a HSV1 tk gene in HBsAg particles (Preparation of HBsAg particles encapsulating HSV1 tk gene)

Next, in order to produce the HBsAg particles as a drug of the present invention encapsulating HSV1 tk gene, a cancer-treating thymidine kinase derived from simple herpes virus (HSV1 tk) was sealed in the HBsAg particles that were prepared according to the described method.

The cancer cells that have incorporated the HSV tk gene become ganciclovir (GCV) sensitive when they express the gene. Administration of ganciclovir therefore kills off the cancer cells by the strong effect it exhibits on the cancer cells. This is one reason the HSV tk gene has been widely used in the gene therapy of cancer.

In this Example, the HSV tk gene was sealed in the HBsAg particles using a vector pGT65-hIFN-α (the product of Invitrogen Corporation) that expresses the HSV tk gene. The HBsAg particles encapsulating the HSV tk gene were obtained by transferring the expression vector into the HBsAg particles by an electroporation method. Specifically, 10 µg of expression vector was transferred into 50 µg of L protein particles in the HBsAg particles. The vector was transferred using a PBS buffer, and the electroporation was carried out with a 4 mm cuvette under 220 V and 950 µF.

### [Example D] Effectiveness of cancer treatment using the HBsAg particles encapsulating HSV1 tk gene with respect to nude rats to which human hepatic carcinoma is transplanted

Next, the effectiveness of the treatment of hepatic cancer using the HBsAg particles encapsulating HSV1 tk gene prepared according to the described method was confirmed on laboratory animals.

As the laboratory animal, the present Example used nude rats purchased from CLEA Japan, Inc. (lineage: F344/NJcl-rnu/rnu, female). The effectiveness of the treatment was confirmed on cancer-bearing rats prepared by transplanting into the bilateral dorsal area of the nude rats, either human hepatic cancer-derived cells HuH-7 (JCRB0403), or the human colon cancer-derived cells WiDr (ATCC CCL-218) as a negative control. The tumor cells were respectively mixed with Matrigel (product of Beckton, Dickinson and Company) and used according to the instructions, before being grafted to the nude rats. The cancer-bearing nude rats were grown for about 3 weeks until the grafted tumor developed into a solid cancer tumor of about 2 to 3 cm diameter.

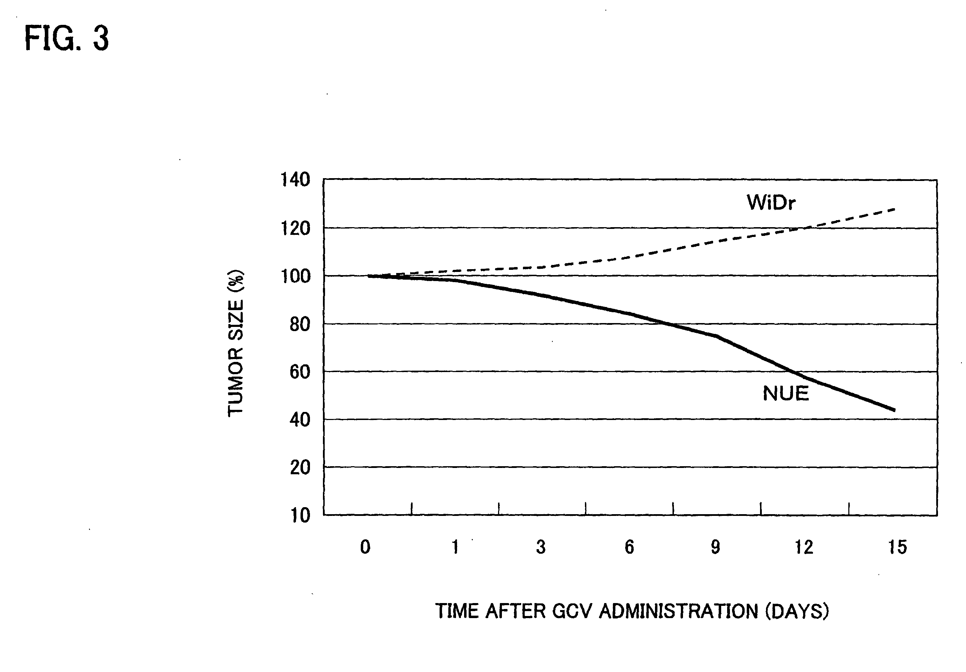

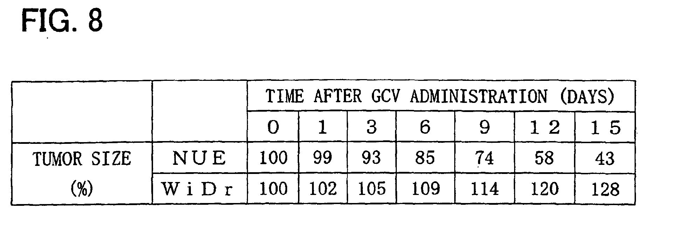

Approximately 10 µg of HBsAg particles encapsulating the HSV1 tk gene were administered to each cancer-bearing rat through the tail vein (intravenous injection). Starting from 5 days after the intravenous injection, ganciclovir (GCV) was administered to each cancer-bearing rat with the dose of 50 mg/kg/day, using an osmotic pump (alzet osmotic pump; Cat No. 2ML2). Here, the GCV was administered to the back of each cancer-bearing rat subcutaneously. The GCV was administered for no longer than 14 days. After the administration, the state (size) of the tumor tissue of the cancer-bearing rats was observed over time. Specifically, the major axis and minor axis of the tumor part were measured with a gauge, and a tumor volume was approximated (major axis × minor axis × minor axis/2). The rats were measured in triplet. The results are shown in Fig. 3 and Fig. 8.

As shown in Fig. 3 and Fig. 8, retraction of the human colon cancer-derived tumor tissue (WiDr) over time was not observed, and the effectiveness of the treatment was not confirmed. In contract, retraction of the human hepatic cancer-derived tumor cells (NUE) over time was observed, and the therapeutic effect specific to hepatic cancer using the HBsAg-HSV1 tk particles was confirmed.

As described above, with the HBsAg-HSV1 tk particles as a drug of the present invention, it was confirmed that the transfer of the gene was very specific and efficient in the human hepatic cells and therefore highly effective in hepatic cancer treatment. Further, the present experiment established on the laboratory animal level, the protocol of the treatment of hepatic cancer using the HBsAg-HSV1 tk particles.

The invention being thus described, it will be obvious that the same may be varied in many ways. Such variations are not to be regarded as a departure from the spirit and scope of the invention, and all such modifications as would be obvious to one skilled in the art are intended to be included within the scope of the following claims.

### INDUSTRIAL APPLICABILITY

As described above, the present invention provides a drug that can be used by a convenient method of intravenous injection to specifically and effectively treat hepatic diseases typified by hepatic carcinoma. The invention is a great leap forward from conventional gene therapy in that it does not require any major surgery, and that the risk of side effect is greatly reduced. The drug is therefore usable in clinical applications in its present form.

## Claims

1. A drug that comprises hollow nanoparticles of a particle-forming protein, the hollow nanoparticles having an ability to recognize a hepatocyte, and encapsulating a substance to be transferred to a cell for treatment of a hepatic disease.

2. The drug as set forth in claim 1, wherein the protein comprises a hepatitis B virus surface-antigen protein.

3. The drug as set forth in claim 1 or 2, wherein the hepatic disease-treating substance comprises a gene.

4. The drug as set forth in claim 3, wherein the gene comprises a cancer-treating gene.

5. The drug as set forth in claim 4, wherein the gene comprises a thymidine kinase (HSV1tk) gene derived from simple herpes virus.

6. The drug as set forth in any one of claims 1 through 5, wherein the drug is administered to the human body through intravenous injection.

7. A hepatic disease treating method comprising administering the drug of any one of claims 1 through 6.
